Europäisches Patentamt

European Patent Office

Office européen des brevets

(11) Veröffentlichungsnummer: **0 518 897 B1**

# EUROPÄISCHE PATENTSCHRIFT

(45) Veröffentlichungstag der Patentschrift: **19.04.95**

(51) Int. Cl.[6]: **A61K 7/16**

(21) Anmeldenummer: **91904641.7**

(22) Anmeldetag: **28.02.91**

(86) Internationale Anmeldenummer:
**PCT/EP91/00372**

(87) Internationale Veröffentlichungsnummer:
**WO 91/13607 (19.09.91 91/22)**

(54) **DEN ZAHNSCHMELZ SCHÜTZENDE MUND- UND ZAHNPFLEGEMITTEL.**

(30) Priorität: **09.03.90 DE 4007431**

(43) Veröffentlichungstag der Anmeldung:
**23.12.92 Patentblatt 92/52**

(45) Bekanntmachung des Hinweises auf die
Patenterteilung:
**19.04.95 Patentblatt 95/16**

(84) Benannte Vertragsstaaten:
**AT BE CH DE DK ES FR GB IT LI LU NL SE**

(56) Entgegenhaltungen:
**EP-A- 0 166 055**
**WO-A-82/03008**
**WO-A-87/07615**

**Dialog Information Services, Datenbank 155 :**
**Medline A.N.=03074176 D.B. Boyer et al "Re-**
**mineralization of dentin in vitro"**

(73) Patentinhaber: **Henkel Kommanditgesellschaft**
**auf Aktien**

**D-40191 Düsseldorf (DE)**

(72) Erfinder: **WÜLKNITZ, Peter**
**Am Alten Broich 69**
**D-4018 Langenfeld (DE)**
Erfinder: **LASKA, Hans**
**Sperberstrasse 10**
**D-4000 Düsseldorf (DE)**
Erfinder: **PLÖGER, Walter**
**Holbeinweg 11**
**D-4010 Hilden (DE)**

EP 0 518 897 B1

**Beschreibung**

Die Erfindung betrifft Mund- und Zahnpflegemittel, die zum Schutz gegen eine Demineralisation des Zahnschmelzes eine Kombination von wasserlöslichen Fluorverbindungen und Phosvitin enthalten.

Mund- und Zahnpflegemittel sind Produkte, die der Reinigung und Pflege der Mundhöhle, der Zähne und des Rachenraumes dienen. Die Aufgabe von Mund- und Zahnpflegemitteln ist neben der Beseitigung von Mundgeruch und der Reinigung der Zähne von Zahnbelägen die Vorbeugung vor Zahnerkrankungen wie Karies.

Eine an sich bekannte Strategie besteht darin, den Zahnschmelz gegen Angriffe zu schützen oder widerstandsfähiger zu machen. Es ist z. B. bekannt, daß ein aus den Bestandteilen des Speichels gebildeter dünner Proteinfilm auf den Zähnen, die sog. Pellicle, diese vor übermäßigem Mineralverlust schützt. Es ist auch bekannt, daß dieser Proteinfilm aus Phosphoproteinen besteht, die an Serin gebundene Phosphorsäuregruppen tragen und eine besonders hohe Haftung an der Schmelzoberfläche besitzen. Aus WO82/03008 war auch bereits bekannt, daß solche Phosphorproteine Zahnpflegemitteln zugesetzt werden können und durch ihren Zusatz die Auflösung des Hydroxylapatits, des Hauptbestandteils von Zahnschmelz, verringert wird.

Es ist auch seit langem üblich, Fluor in Form von wasserlöslichen Fluoriden oder Monofluorphosphaten den Mund- und Zahnpflegemitteln zuzusetzen, um auf diese Weise die Widerstandsfähigkeit des Zahnschmelzes vor den kariösen Angriff zu erhöhen.

Es wurde nun überraschend festgestellt, daß die schmelzfestigende und Demineralisierung hemmende Wirkung des Fluors bereits durch kleine Zusätze an Phosvitin noch erheblich gesteigert werden kann. Dies ist so erstaunlich, da mit Phosphoproteinen alleine nur wesentlich geringere Effekte erzielt werden können als mit Fluorid.

Gegenstand der Erfindung sind daher Mund- und Zahnpflegemittel in Form von wässrigen, wässrig-alkoholischen oder wasserfreien Zubereitungen, die zum Schutz gegen eine Demineralisierung des Zahnschmelzes eine Kombination von Fluorid oder Monofluorphosphat und Phosvitin oder dessen wasserlöslichen Salzen in einer Menge enthalten, daß bei der Anwendung eine Konzentration von 0,01 - 0,2 Gew.-% gelöstem Fluor und 0,01 - 0,4 Gew.-% Phosvitin vorliegt.

Mund- und Zahnpflegemittel in Sinne der vorliegenden Erfindung sind Zahnpulver, Zahnpasten, Zahngele, Zahncremes, Mundwässer, Mundspülungen, Lutschtabletten und Kaugummis.

Um die erfindungsgemäße Mindestkonzentration an gelöstem Fluor und Phosvitin in der Mundhöhle zu gewährleisten, ist erforderlich, daß die unverdünnt angewendeten Zubereitungen wie z. B. Zahnpasta, Zahngele, Zahncremes, Zahnpulver und Mundwässer 0,01 - 0,2 Gew.-% Fluor in Form eines wasserlöslichen Fluorids oder Monofluorphosphats und 0,01 - 0,4 Gew.-% Phosvitin enthalten. Dabei ist eine Verdünnung durch den Speichel und das beim Zähneputzen verwendete Wasser berücksichtigt.

Mundwasserkonzentrate sollten eine dem vorgesehenen Verdünnungsverhältnis entsprechende, höhere Konzentration an gelöstem Fluor und Phosvitin enthalten. Mund- und Zahnpflegemittel in Form von Lutschtabletten oder Kaugummi, deren Wirkstoffe während der Anwendung um ein Mehrfaches mit Speichel verdünnt werden, sollten etwa 0,05 - 1 Gew.-% Fluor in Form von wasserlöslichem Fluorid oder Monofluorphosphat und 0,1 - 2 Gew.-% Phosvitin enthalten.

Als wasserlösliche Fluorverbindungen können Alkalifluoride, z. B. Natriumfluorid, Magnesiumfluorid, Zinkfluorid und Zinnfluorid, aber auch Fluoride von Aminoverbindungen und oberflächenaktive quartäre Ammoniumfluoride enthalten sein. Auch können Alkali-Monofluorphosphate, z. B. Natriummonofluorphosphat, eingesetzt werden.

Phosvitin ist ein Glycophosphoprotein, welches aus dem Eigelb von Vogel- und Amphibien-Eiern und aus Fischrogen nach bekannten Verfahren isoliert wird. Es gibt verschieden Typen von Phosvitinen, wobei aus Hühnereiern eine Phosvitin-Hauptkomponente mit einem Molekulargewicht von 34000 - 36000 Dalton und eine kleinere Komponente mit einem Molekulargewicht von ca. 28000 Dalton erhältlich ist. Kennzeichnend für alle Phosvitine ist ein hoher Gehalt an phosphatiertem Serin (Phosphoserin). Bei einem Gehalt von circa 119 Phosphoserin-Bausteinen (mehr als die Hälfte der AminosäureBausteine) pro Molekül weist das Phosvitin einen Phosphorgehalt von ca. 10 Gew.-% auf und stellt daher das am höchsten phosphatierte natürliche Protein dar. Phosvitin ist im Handel erhältlich. Phosvitin kann auch in Form eines wasserlöslichen Salzes, z. B. als Natrium- oder Ammoniumsalz verwendet werden.

Die erfindungsgemäßen Mund- und Zahnpflegemittel enthalten die kennzeichnende Kombination von gelöstem Fluor und Phosvitin in einem für die jeweilige Zubereitungsform üblichen Träger.

Bei Mundwässern besteht dieser Träger im wesentlichen aus Wasser, Ethanol, ätherischen Ölen, Emulgatoren und Lösungsvermittlern, Geschmackskorrigenten (z. B. Süßstoff), sowie gegebenenfalls adstringierenden oder tonisierenden Drogenauszügen, Farbstoffen und Konservierungsstoffen. Besonders gut

geeignete Emulgatoren und Lösungsvermittler sind ethoxylierte Sorbitanfettsäureester, ethoxylierte Glycerinfettsäureester, Alkylglucoside, Fettsäurepolyglycoester oder Ethylenoxid-Propylenoxid-Blockpolymerisate.

Bevorzugt enthalten erfindungsgemäße Mund- und Zahnpflegemittel in Form eines Mundwassers in der Anwendungskonzentration 1 - 10 Gew.-% Ethanol.

Unter Zahnpasten, Zahncremes oder Zahngelen werden im allgemeinen pastöse Zubereitungen aus Wasser, Konsistenzreglern, Feuchthaltemitteln, Schleif- oder Putzkörpern, Tensiden, Süßungsmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden. Bevorzugt sind aber erfindungsgemäße Mund- und Zahnpflegemittel in Form einer Schleif- und Putzkörper enthaltenden Zubereitung oder eines Gels, die als Schleif- und Putzkörperkomponente Kieselsäuren oder Aluminiumoxid-trihydrat enthalten. Diese Schleif- und Putzkörper-Komponente macht bevorzugt 15 - 50 Gew.-% der Zahnpasta aus.

Als Feuchthaltemittel können z. B. Glycerin, Sorbit, Xylit, Propylenglycole, Polyethylenglycole, insbesondere solche mit mittleren Molekulargewichten von 200 - 800 eingesetzt werden. Als Konsistenzregler (bzw. Bindemittel) dienen z. B. natürliche und/oder synthetische wasserlösliche Polymere wie Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthangum, Johannisbrotmehl, Pectine, wasserlösliche Carboxyvinypolymere (z. B. Carbopol®-Typen), Polyvinylalkohol, Polyvinylpyrrolidon, und Polyethylenglycole, insbesondere solche mit Molekulargewichten von 1 500 - 1 000 000.

Weitere Stoffe, die sich zur Viskositätskontrolle eignen, sind z. B. Schichtsilikate wie z. B. Montmorillonit-Tone, kolloidale Verdickungskieselsäure wie z. B. Aerogel-Kieselsäure oder pyrogene Kieselsäuren.

Als Tenside eignen sich alle wasserlöslichen, anionischen, kationischen, ampholytischen, zwitterionischen und nichtionischen Tenside. Bevorzugt sind die bereits genannten nichtionischen Tenside, insbesondere die ethoxylierten Sorbitanfettsäureester, die ethoxylierten Glycerinfettsäureester, z. B. Rizinusöl-Ethoxylate, die Alkylglucoside, Fettsäurepolyglycolester und Ethylenoxyd-Propylenoxid-Mischpolymerisate.

Weitere übliche Zahnpastenzusätze sind
- Konservierungsmittel und antimikrobielle Stoffe, wie z. B.
- p-Hydroxybenzoesäuremethyl-, -ethyl oder -propylester, Natriumsorbat, Natriumbenzoat, Bromchlorophen, Phenyl-salicylsäureester, Thymol usw.
- Antizahnsteinwirkstoffe, z. B. Organophosphonate wie die Natriumsalze von 1-Hydroxyethan-1,1-diphosphonsäure, Azacycloheptan-1,1-diphosphonsäure, 1-Phosphonopropan-1,2,3-tricarbonsäure und andere Phosphonsäuren wie sie z. B. aus US-PS 3 488 419, DE-OS 22 24 430 und DE-OS 23 43 196 bekannt sind,
- Süßungsmittel wie z. B. Saccharin-Natrium, Natrium-Cyclamat, Sucrose, Lactose, Maltose, Fructose,
- Aromen wie z. B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,
- Pigmente wie z. B. Titandioxid,
- Farbstoffe,
- Puffersubstanzen wie z. B. primäre, sekundäre oder tertiäre Alkaliphosphate oder Citronensäure/Natriumcitrat,
- Wundheilende und entzündungshemmende Stoffe wie z. B. Allantoin, Harnstoff sowie Azulen, Kamillenwirkstoffe, Acetylsalicylsäurederivate.

Die folgenden Beispiele sollen den Erfindungsgegenstand näher erläutern:

Beispiele

Prüfung der Zahnschmelz-Demineralisation

## 1. Prüfmethode

Das Prinzip der Prüfung beruht auf der Beobachtung, daß durch Demineralisation die Festigkeit des Zahnschmelzes verringert wird. Dieser Festigkeitsverlust läßt sich mit dem Verfahren der Eindruck-Härtemessung erfassen. Das Meßprinzip beruht auf der Messung der Eindringtiefe eines Diamanten mit definierter Geometrie, der mit einem bestimmten Auflagegewicht senkrecht in das Testmaterial eingedrückt wird. Verwendet wurde das Verfahren nach KNOOP, das einen rautenförmigen Eindruck im Material hinterläßt. (Knoop, F., Peters, C.G., Emerson, W.B., A sensitive pyramidaldiamond tool for indentation measurements. J.Res.natn. Bur.Stand. 23, 39-61 (1939)). Je höher die Eindringtiefe, desto stärker ist die Demineralisierung fortgeschritten. Bei dem verwendeten vitro-Verfahren werden die natürlichen De- und Remineralisierungsvorgänge an Rinderschmelz nachgestellt.

2. Methodik

2.1 **Vorbereitung der Zahnschmelzblöcke**

Die Vorderseiten von Rinder-Schneidezähnen werden in Stücke von 3 x 5 mm Größe (Slabs) geschnitten, und mit Wachs so auf Plexiglas-Würfeln (1 x 2,5 x 2,5 cm) montiert, daß nur die Schmelzoberfläche frei bleibt. Die Schmelzoberfläche wird poliert, bis sie einheitlich glatt erscheint. Pro Testgruppe werden 4 Slabs verwendet.

2.2 **Behandlungen**

Das in vitro-Testverfahren, das zur Prüfung verwendet wurde, beginnt mit einer 7-stündigen Demineralisierungsphase (Milchsäure-Lösung) gefolgt von einer 16-stündigen Remineralisierungsphase (Remineralisierungslösung). Diese Behandlungsschritte werden dreimal wiederholt. Das Phosvitin ist in beiden Lösungen in der jeweils angegebenen Konzentration enthalten. Wird Fluorid bei einer Testgruppe verwendet, appliziert man es durch jeweils 5-minütiges Eintauchen der Slabs in NaF-Lösung der in Tabelle I angegebenen Konzentration vor jeder einzelnen Behandlungsphase.

**Demineralisierungslösung**

100 ml 1M Milchsäure und 500 mg Hydroxylapatit wurden mit NaOH-Lösung auf einen pH von 4,6 eingestellt und nach Zugabe der in Tabelle I angegebenen Menge Phosvitin mit Aqua dest. auf 1 l aufgefüllt.

**Remineralisierungslösung (künst. Speichel)**

654 mg KH2PO4,
825,5 mg Na2HPO4x2H2O,
1168 mg NaCl,
2534 mg KCl und
540 mg CaCl2x2H2O
wurden in Aqua dest. gelöst, mit 1M NaOH oder HCl-Lösung auf pH 6,5 eingestellt und nach Zusatz der in Tabelle I angegebenen Menge Phosvitin auf 2 l mit Aqua dest. aufgefüllt.

2.3 **Eindruckhärte-Messungen**

Die Messung erfolgte mit dem KNOOP-Diamanten bei einem Auflagegewicht von 55 g am unbehandelten Zahn und nach jeder Behandlungsphase. Angegeben wird die Länge der großen Eindruck-Diagonale in μm.

2.4 **Berechnung der Daten**

Für jeden einzelnen Slab wurden die Differenzen zwischen der Eindrucklänge nach den einzelnen Behandlungen und vor Beginn der Behandlungen bestimmt. Die jeweiligen Mittelwerte der Testgruppen zu 4 Slabs wurden dann zu dem Mittelwert der Kontrollgruppe (ohne Protein und Fluorid) in Bezug gesetzt nach folgender Formel:

$$SDR = \frac{\text{Differenz Eindrucklänge Testgruppe}}{\text{Differenz Eindrucklänge Kontrollgruppe}} \times 100$$

4

Der Wert SDR (Schmelz-Demineralisierung-Reduktion) gibt somit die prozentuale Verringerung der Demineralisierung im Vergleich zur Kontrolle wieder.

## 3. **Ergebnisse**

Die Werte der Schmelz-Demineralisierungs-Reduktion sind für die einzelnen Testgruppen in der Reihenfolge der Demineralisierungs bzw. Remineralisierungs-Schritte angeordnet angegeben.

Tabelle I zeigt, wie durch Behandlung mit 250 ppm gelöstem Fluor (0,055 Gew.-% NAF) bei den mehrfachen De- und auch Remineralisierungsschritten starke Schutzeffekte im Vergleich zur Kontrolle auftreten.

Bei der Verwendung von nur 0,02 Gew.-% Phosvitin sind diese Effekte vergleichsweise gering, mit 0,1 Gew.-% Phosvitin deutlich stärker, aber größtenteils geringer als mit Fluorid allein.

Die Kombination der Behandlung mit Phosvitin und Fluorid läßt nun einen starken zusätzlichen Schutzeffekt erkennen, der sogar bei der niedrigen Phosvitin-Konzentration den guten Fluorid-Effekt deutlich übertrifft. Durch die kombinierte Verwendung von Phosvitin und Fluorid wird der Zahn nahezu unempfindlich gegen Demineralisierung. Da Phosvitin aufgrund seiner höheren Zahl an Haftgruppen den Pellicleproteinen überlegen ist, sind aus der Verwendung der erfindungsgemäßen Kombination mit antikariesaktiven Fluoriden in allen Mund- und Zahnpflegemitteln Vorteile zu sehen.

Tabelle I

| Gruppe | 1 | 2 | 3 | 4 | 5 |
|---|---|---|---|---|---|
| NaF (Gew.-%) | 0,055 | - | - | 0,055 | 0,055 |
| Phosvitin (Gew.-%) | -SDR | 0,02 SDR | 0,1 SDR | 0,02 SDR | 0,1 SDR |
| 7 Std. Demin. | 16 | 29 | 54 | 58 | 84 |
| 16 Std. Remin. | 18 | 27 | 33 | 69 | 74 |
| 7 Std. Demin. | 47 | 22 | 61 | 72 | 89 |
| 16 Std. Remin. | 40 | 17 | 48 | 78 | 85 |
| 7 Std. Demin. | 65 | 33 | 60 | 84 | 91 |
| 16 Std. Remin. | 71 | 35 | 55 | 86 | 88 |
| 7 Std. Demin. | 76 | 19 | 62 | 88 | 91 |
| 16 Std. Remin. | 81 | 28 | 59 | 92 | 92 |

## 4. Anwendungsbeispiele

| 4.1 **Mundwasser I** | |
|---|---|
| Ethanol (99 %, unvergällt) | 7,5 Gew.-% |
| Phosvitin (SIGMA) | 0,4 Gew.-% |
| Natriumfluorid | 0,22 Gew.-% |
| HR 60[1] | 0,25 Gew.-% |
| Aromaöl (Pfefferminz) | 0,2 Gew.-% |
| Aspartam | 0,1 Gew.-% |
| Farbstoff blau CI.42090 (1 % in $H_2O$) | 0,1 Gew.-% |
| PHB-Methylester | 0,1 Gew.-% |
| PHB-Propylester | 0,02 Gew.-% |
| Wasser | ad 100,00 Gew.-% |

[1] Anlagerungsprodukt von 60 Mol Ethylenoxid an gehärtetes Rizinusöl

| 4.2 **Mundwasser II** | |
| --- | --- |
| Ethanol (99 % unvergällt) | 5,0 Gew.-% |
| Phosvitin (SIGMA) | 0,1 Gew.-% |
| Natriummonofluorphosphat | 1,2 Gew.-% |
| HR 60 [1] | 0,5 Gew.-% |
| Aromaöl (Pfefferminz) | 0,3 Gew.-% |
| Aspartam | 0,1 Gew.-% |
| Farbstoff rot CI.16255 (1 % in $H_2O$) | 0,1 Gew.-% |
| PHB-Methylester | 0,1 Gew.-% |
| PHB-Propylester | 0,02 Gew.-% |
| Wasser | ad 100,00 Gew.-% |

[1] Anlagerungsprodukt von 60 Mol Ethylenoxid an gehärtetes Rizinusöl

**Patentansprüche**

1. Mund- und Zahnpflegemittel in Form von wässrigen, wässrig-alkoholischen oder wasserfreien Zubereitungen, dadurch gekennzeichnet, daß sie zum Schutz gegen eine Demineralisierung des Zahnschmelzes eine Kombination von Fluorid oder Monofluorphosphat und Phosvitin oder dessen löslichen Salzen in einer Menge enthalten, daß bei der Anwendung eine Konzentration von 0,01 - 0,2 Gew.-% gelöstem Fluor und 0,01 - 0,4 Gew.-% Phosvitin vorliegt.

2. Mund- und Zahnpflegemittel nach Anspruch 1 in Form einer Schleif- und Putzkörper enthaltenden Zahnpasta oder eines Gels, dadurch gekennzeichnet, daß als Schleif- und Putzkörper-Komponente Kieselsäuren oder Aluminiumoxid-trihydrat enthalten sind.

3. Mund- und Zahnpflegemittel nach Anspruch 1 in Form eines Mundwassers, dadurch gekennzeichnet, daß dieses bei der Anwendungskonzentration 1 - 10 Gew.-% Ethanol enthält.

**Claims**

1. Oral and dental hygiene preparations in the form of aqueous, aqueous-alcoholic or water-free compositions, characterized in that, for protection against demineralization of enamel, they contain a combination of fluoride or monofluorophosphate and phosvitin or soluble salts thereof in such a quantity that, on application, a concentration of 0.01 to 0.2% by weight of dissolved fluorine and 0.01 to 0.4% by weight of phosvitin is present.

2. Oral and dental hygiene preparations as claimed in claim 1 in the form of a toothpaste containing abrasives or polishes or a gel, characterized in that silicas or trihydrated aluminium oxide are present as the abrasive and polishing component.

3. Oral and dental hygiene preparations as claimed in claim 1 in the form of a mouthwash, characterized in that, in its in-use concentration, the mouthwash contains 1 to 10% by weight of ethanol.

**Revendications**

1. Produits d'hygiène bucco-dentaire sous la forme de préparations aqueuses, aqueuses-alcooliques ou anhydres, caractérisés en ce qu'ils renferment, pour protéger l'émail de la déminéralisation une association de fluorure ou de monofluorophosphate et de phosvitine ou de ses sels solubles dans l'eau, dans une proportion telle qu'on obtient lors de l'utilisation une concentration de 0,01 à 0,2 % en poids de fluor dissous et de 0,01 à 0,4 % en poids de phosvitine.

2. Produits d'hygiène bucco-dentaire selon la revendication 1, sous la forme d'un dentifrice ou d'un gel renfermant des abrasifs ou des corps nettoyants, caractérisés en ce qu'ils contiennent comme composant abrasifs ou corps nettoyants, des acides siliciques ou de l'oxyde d'aluminium trihydraté.

3. Produits d'hygiène bucco-dentaire selon la revendication 1, sous la forme d'une eau dentifrice, caractérisés en ce que celle-ci renferme 1 à 10 % en poids d'éthanol à la concentration d'utilisation.